(19) Europäisches Patentamt
European Patent Office
Office européen des brevets

(11) Veröffentlichungsnummer : **0 046 445**
**B1**

(12) **EUROPÄISCHE PATENTSCHRIFT**

(45) Veröffentlichungstag der Patentschrift : **13.06.84**

(21) Anmeldenummer : **81810319.4**

(22) Anmeldetag : **10.08.81**

(51) Int. Cl.³ : **C 07 C123/00**, C 07 C119/18, C 07 D295/18, A 01 N 37/52, A 01 N 43/84, A 01 N 43/40

(54) **3-Phenoxy-methylenaniline als Herbizide, ihre Herstellung und Verwendung.**

(30) Priorität : **15.08.80 CH 6198/80**

(43) Veröffentlichungstag der Anmeldung : **24.02.82 Patentblatt 82/08**

(45) Bekanntmachung des Hinweises auf die Patenterteilung : **13.06.84 Patentblatt 84/24**

(84) Benannte Vertragsstaaten : **BE CH DE FR GB LI NL**

(56) Entgegenhaltungen :
**EP-A- 0 007 482**
**DE-A- 1 542 715**
**DE-A- 1 542 854**
**DE-A- 2 944 783**
**GB-A- 1 521 472**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Dürr, Dieter, Dr.**
**Brändelistalweg 16**
**CH-4103 Bottmingen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 046 445 B1

**Beschreibung**

Die vorliegende Erfindung betrifft neue, herbizid wirksame 3-Phenoxy-methylenaniline, Verfahren zu ihrer Herstellung, sie als Wirkstoffe enthaltende herbizide Mittel, sowie deren Verwendung zur Bekämpfung von Unkräutern, vor allem selektiv in Nutzpflanzenkulturen.

Die erfindungsgemässen 3-Phenoxy-methylenaniline entsprechen der allgemeinen Formel I

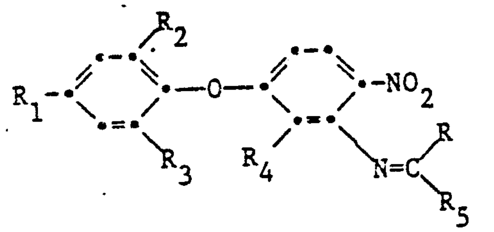

(I)

worin

R einen Rest

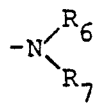

oder $C_1$-$C_4$-Alkoxy,

$R_1$ Halogen oder $C_1$-$C_2$-Halogenalkyl,

$R_2$, $R_3$ und $R_4$ unabhängig voneinander je Wasserstoff oder Halogen,

$R_5$ Wasserstoff oder $C_1$-$C_4$-Alkyl,

$R_6$ Wasserstoff, $C_1$-$C_4$-Alkyl, $C_2$-$C_4$-Alkenyl oder $C_2$-$C_4$-Alkinyl und

$R_7$ Wasserstoff oder $C_1$-$C_4$-Alkyl bedeuten, wobei die Reste $R_5$ und $R_6$ oder $R_6$ und $R_7$ zusammen mit dem Stickstoffatom einen gegebenenfalls durch weitere Heteroatome unterbrochenen Heterocyclus bilden können.

Die Formel I umfasst auch die Salze, die die Amidine (R in Formel I bedeutet dann —$NR_6R_7$) mit Säuren bilden können.

Verschiedene Formamidine mit mikrobiziden, ektoparasitiziden, insektiziden oder fungiziden Eigenschaften sind in den japanischen Patentveröffentlichungen J 4 801 123 und J 7 249 691 und den französischen Patentschriften N° 1 353 726 und 1 504 840 beschrieben worden. Formamidine mit herbiziden Eigenschaften sind aus der europäischen Patentveröffentlichung EP-A-7482 bekannt.

In den Definitionen von $R_1$ bis $R_4$ sind unter Halogen, Fluor, Chlor oder Brom, vorzugsweise jedoch Fluor und insbesondere Chlor zu verstehen.

Unter Alkyl sind geradkettige oder verzweigte Alkylreste zu verstehen, wie beispielsweise Methyl, Aethyl, n-Propyl, i-Propyl oder die vier isomeren Butylreste; vorzugsweise sind die Reste jedoch geradkettig.

Die Alkoxyreste in der Definition von R sind geradkettige Reste wie Methoxy, Aethoxy, n-Propyloxy und n-Butyloxy.

Sinngemäss sind unter Halogenalkyl im allgemeinen zu verstehen: Fluormethyl, Chlormethyl, α-Chloräthyl, β-Chloräthyl, Trifluormethyl, oder, α,α,β,β-Tetrachloräthyl, vor allem aber Chlormethyl und Trifluormethyl.

Beispiele für Alkenyl sind Vinyl, Allyl, Methallyl und Crotyl, insbesondere aber Vinyl und Allyl.

Beispiele für Alkinyl sind im gleichen Sinn Aethinyl und insbesondere Propargyl.

Beispiele für mögliche Stickstoffheterocyclen sind Pyrrolidin, Pyrrolin, Imidazolidin, Pyrazolidin, Piperidin, Piperazin, Morpholin, Pyrazolin, Imidazol, Pyrrol oder Imidazolin, vorzugsweise aber Pyrrolidin, Piperidin und Morpholin.

Beispiele für Säuren, die mit den Amidinen Salze bilden können, sind anorganische Säuren wie Salzsäure, Bromwasserstoffsäure, Schwefelsäure, Phosphorsäure oder organische Säuren, wie Sulfonsäuren z. B. Toluolsulfonsäure oder gegebenenfalls halogenierte Mono- oder Dicarbonsäuren z. B. Ameisensäure, Essigsäure, Trichloressigsäure, Trifluoressigsäure, Oxalsäure und Methansulfonsäure oder Phthalsäure.

Wegen ihrer Wirkung bevorzugt sind die Verbindungen der Formel I, in denen der Rest R die Aminogruppe —$NR_6R_7$ darstellt.

Weiter bevorzugt sind die Wirkstoffe der Formel I, in denen $R_1$ die Trifluormethylgruppe bedeutet.

Eine besonders günstige selektive Wirkung haben die Verbindungen der Formel I, in denen von den Resten $R_2$, $R_3$ und $R_4$ unabhängig voneinander jeweils 1 bis 2 Reste Chloratome darstellen und die übrigen Wasserstoff bedeuten.

Besonders bevorzugt sind die Verbindungen der Formel I, in denen $R_1$ Trifluormethyl, $R_2$ Chlor und $R_3$ und $R_4$ Wasserstoff bedeutet oder in denen einer der Reste $R_3$ und $R_4$ Chlor und der andere Wasserstoff ist.

Die Herstellung der 3-Phenoxy-methylenaniline der Formel I erfolgt nach an sich bekannten Methoden.

Nach einem ersten Verfahren werden die Verbindungen der Unterformel Ia

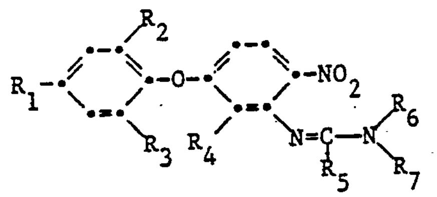

(Ia)

hergestellt, indem man ein Nitroanilin der Formel II

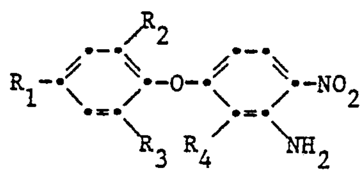

(II)

in Gegenwart eines Kondensationsmittels und gegebenenfalls einer Base mit einem Carbamid der Formel III

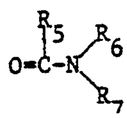

(III)

umsetzt und gegebenenfalls in die Salze überführt. In den Formeln Ia, II und III haben $R_1$ bis $R_7$ die unter Formel I angegebene Bedeutung.

Als Kondensationsmittel kommen beispielsweise Thionylchlorid, Phosgen, Phosphoroxytrichlorid, Phosphorpentachlorid, Phosphortrichlorid, Dimethylsulfat oder p-Toluolsulfonsäurechlorid und als Basen Natrium- und Kaliumhydroxyd, Natriumhydrogencarbonat, Natriumcarbonat, Calciumoxyd oder Calciumcarbonat in Betracht.

Nach einem anderen Verfahren erhält man die Verbindungen der Unterformel Ia, indem man ein Anilin der Formel II mit einem Carbamiddiacetal der Formel IV

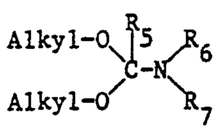

(IV)

worin $R_5$ bis $R_7$ die unter Formel I gegebene Bedeutung haben und Alkyl geradkettiges $C_1$-$C_4$-Alkyl bedeutet, umsetzt und gegebenenfalls in die Salze überführt.

Schliesslich kann man nach einem weiteren Verfahren die Verbindungen der Unterformel Ib

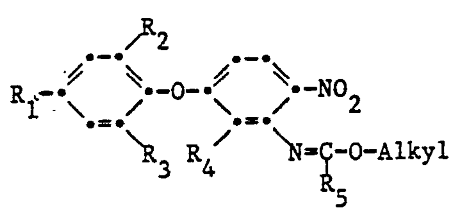

(Ib)

erhalten, indem man ein Anilin der Formel II mit einem Orthoester der Formel V

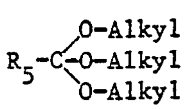

(V)

worin Alkyl und $R_1$ bis $R_5$ die unter Formel IV angegebene Bedeutung haben umsetzt.

Die Verbindungen der Formel Ia lassen sich auch erhalten, indem man einen Iminoäther der Formel Ib mit einem Amin der Formel VI

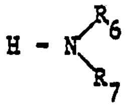

(VI)

umsetzt, worin $R_6$ und $R_7$ die unter Formel I gegebene Bedeutung haben.

Die Salze der Amidine Ia erhält man, indem man die Amidine in einem inerten Lösungsmittel löst, mit äquimolaren Mengen Säure umsetzt und das Lösungsmittel abdampft.

Diese Umsetzungen werden vorteilhafterweise in inerten, organischen Lösungsmitteln vorgenommen wie Methanol, Aethanol, Isopropanol, Methylenchlorid, Chloroform, Tetrahydrofuran, Dioxan, Acetonitril oder Toluol.

Die Wirkstoffe der Formel I sind stabile Verbindungen. Ihre Handhabung bedarf keiner vorsorglicher Massnahmen.

Die Verbindungen der Formel I sind neu und besitzen interessante herbizide und den Pflanzenwuchs regulierende Wirkung. Vor allem zeichnen sie sich durch hervorragende selektiv-herbizide Eigenschaften aus, die sie ausgezeichnet zum Einsatz in Kulturen von Nutzpflanzen wie Reis, Mais, Hirse, Getreide, Baumwolle, Soja, Zuckerrüben, Gemüsekulturen aller Art sowie von Zierpflanzenkulturen, vorzugsweise aber Weizen befähigen.

Die herbizide Wirkung tritt bei den Verbindungen der Formel I im Vergleich zu bekannten Verbindungen schon bei sehr geringen Aufwandmengen ein. Die Aufwandmengen betragen dabei im allgemeinen 0,005 bis 5 kg, vorzugsweise jedoch zwischen 0,25 und 2 kg pro Hektar.

Die Wirkstoffe der Formel I können zur pre- oder postemergenten Bekämpfung von Unkräutern angewendet werden. Vorzugsweise werden die Wirkstoffe oder sie enthaltende Mittel jedoch nach dem Auflaufen der Pflanzen appliziert.

Im weiteren regulieren die Verbindungen der Formel I das Pflanzenwachstum. Sie beeinflussen das Pfanzenwachstum in verschiedener Weise. So hemmen, verzögern oder unterbinden sie in erster Linie das Wachstum und die Keimung. Es handelt sich dabei also sowohl um pre- und post-emergente Herbizidwirkung als auch um Wuchshemmung.

Erfindungsgemässe Mittel, die als aktive Komponente mindestens eine Verbindung der Formel I enthalten, eignen sich besonders zur Hemmung und Kontrolle des Pflanzenwachstums von monocotylen und dicotylen Pflanzen, wie Gräsern, Sträuchern, Bäumen, Getreide- und Leguminosenkulturen, Zuckerrohr, Tabak, Soja, Zwiebel- und Kartoffelknollen, Zierpflanzen, Obstbäumen und Reben.

Die von den neuen Wirkstoffen der Formel I in erster Linie erzielte Wirkung besteht in der gewünschten Reduktion der Pflanzengrösse, insbesondere der Wuchshöhe. Im allgemeinen ist damit eine gewisse Aenderung der Pflanzenform verbunden. In unmittelbarem Zusammenhang zur Verminderung der Wuchshöhe erfährt die Pflanze eine Festigung. Blätter und Stengel sind kräftiger ausgebildet. Durch Verkürzung der Internodienabstände an diversen Pflanzen wird die Knickfestigkeit erhöht. Auf diese Weise können Ernteausfälle durch Gewittersturm, Dauerregen, usw., die normalerweise zu einem Lagern von Getreide- und Leguminosenkulturen führen, weitgehend verhindert und damit die Erntearbeit erleichtert werden. Als Nebeneffekt führt verminderte Wuchshöhe bei Nutzpflanzen zu einer Einsparung an Düngemitteln. In gleichem Masse gilt dies auch für Zierpflanzen, Zierrasen, Sportrasen oder sonstige Grünanpflanzungen.

Eines der wichtigsten Probleme an reinen Grasbepflanzungen ist jedoch der Grasschnitt selbst, sei es an Grünanlagen in Wohngegenden, auf Industriegeländen, auf Sportplätzen, an Autostrassen, Flugpisten, Eisenbahndämmen oder Uferböschungen von Gewässern. In all diesen Fällen ist ein periodisches Schneiden des Rasens bzw. des Grasbewuchses notwendig. Dies ist nicht nur im Hinblick auf Arbeitskräfte und Maschinen sehr aufwendig, sondern bringt im Verkehrsbereich auch erhebliche Gefahren für das betroffene Personal und die Verkehrsteilnehmer mit sich.

Es besteht daher gerade in Gebieten mit grossen Verkehrsnetzen ein dringendes Bedürfnis, die im Hinblick auf die Verfestigung von Seitenstreifen und Böschungen an Verkehrswegen notwendige Grasnarbe einerseits zu erhalten und zu pflegen, andererseits aber mit einfachen Massnahmen während der gesamten Vegetationsperiode auf einer mittleren Wuchshöhe zu halten. Dies wird durch Applikation erfindungsgemässer Wirkstoffe der Formel I auf sehr günstige Weise erreicht.

Die Verbindungen der Formel I vermögen bei gewissen Kulturen, kurz vor der Fruchtreife die Blätter auszutrocknen und zum Fallen zu bringen was dann von Vorteil ist, wenn die betreffende Kultur mangels mechanisch geerntet werden soll. So eignen sich die erfindungsgemässen Verbindungen auch zum Einsatz als Defoliations- und Desikkationsmittel in Baumwoll- und Kartoffelkulturen, kurz vor deren Ernte.

Einige der Verbindungen der Formel I zeigen ausser den herbiziden auch noch insektizide, akarizide, ovizide, ektoparasitizide, nematizide und fungizide Eigenschaften.

Die Erfindung betrifft auch herbizide Mittel, welche einen neuen Wirkstoff der Formel I enthalten, sowie Verfahren zur pre- oder postemergenten Unkrautbekämpfung von mono- und dikotylen Unkräutern, vorzugsweise zweikeimblättrigen, in Nutzpflanzenkulturen wie Mais, Reis, Hirse und Getreide, vor allem in Weizenkulturen.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden :

Feste Aufarbeitungsformen : Stäubemittel, Streumittel, Granulate, Umhüllungsgranulate, Imprägniergranulate und Homogengranulate ;

In Wasser dispergierbare Wirkstoffkonzentrate : Spritzpulver (wettable powder), Pasten Emulsionen, Emulsionskonzentrate und Suspensionskonzentrate (flowable) ;

Flüssige Aufarbeitungsformen : Lösungen.

Der Gehalt an Wirkstoff in den oben beschriebenen Mitteln liegt zwischen 0,1 bis 95 %, bevorzugt zwischen 1 bis 80 %. Anwendungsformen können bis hinab zu 0,001 % verdünnt werden.

Den beschriebenen erfindungsgemässen Mitteln lassen sich andere biozide Wirkstoffe oder Mittel beimischen. So können die neuen Mittel ausser den genannten Verbindungen der allgemeinen Formel I z. B. Insektizide, Fungizide, Bakterizide, Fungistatika, Bakteriostatika, Nematizide oder weitere Herbizide zur Verbreiterung des Wirkungsspektrum enthalten.

In nachfolgenden Beispielen sind Temperaturen in Celsiusgraden angegeben. Teile und Prozentangaben beziehen sich auf das Gewicht, Druckangaben beziehen sich auf Millibar (mb) (1 mb = 100 Pascal).

Beispiel 1

N-[3-(2'-Chlor-4'-trifluormethyl-phenoxy)-6-nitro-phenyl]-N',N'-dimethyl-formamidin.

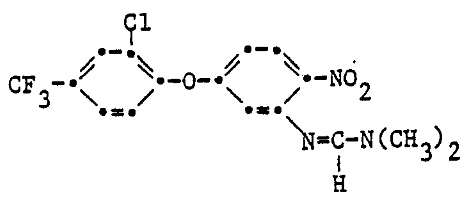

a) 25,2 g 2-Chlor-4-trifluormethyl-3',4'-dinitro-diphenyläther werden in einem Gemisch aus 240 ml Acetonitril und 9 g 30 %-iger Ammoniak-Lösung im Autoklaven für 1 Stunde auf 150 °C erhitzt. Durch Eindampfen der Lösung und Umkristallisieren des Rückstandes erhält man 21 g 3-(2'-Chlor-4'-trifluormethyl-phenoxy)-6-nitro-anilin vom Schmelzpunkt 86-92 °C.

b) 16,5 g 3-(2'-Chlor-4'-trifluormethyl-phenoxy)-6-nitro-anilin und 8 g Dimethylformamid-diäthylacetal werden in 50 ml absolutem Alkohol für 6 Stunden an Rückfluss gekocht und anschliessend eingedampft. Die Kugelrohrdestillation des Rückstandes bei 230 °C/0,09 mb ergibt 18 g N-[3-(2'-Chlor-4'-trifluormethyl-phenoxy)-6-nitrophenyl]-N',N'-dimethylformamidin als viskoses gelbes Oel (Verbindung 1).

Die Verbindung 1 und analog hergestellte Verbindungen sind in der folgenden Tabelle aufgeführt.

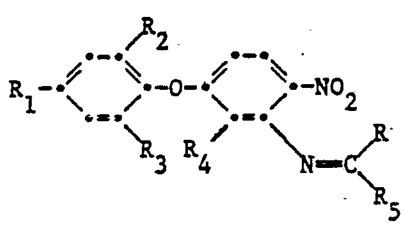

| Nr. | R | $R_1$ | $R_2$ | $R_3$ | $R_4$ | $R_5$ | phys. Daten |
|---|---|---|---|---|---|---|---|
| 1 | $-N(CH_3)_2$ | $CF_3$ | Cl | H | H | H | Sdp. 230°/ 0,09 mb. |
| 2 | $-N(CH_3)_2$ | $CF_3$ | Cl | H | H | $CH_3$ | Sdp. 225°/ 0,03 mb. |
| 3 | 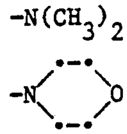 | $CF_3$ | Cl | H | H | H | |

(Fortsetzung)

| Nr. | R | $R_1$ | $R_2$ | $R_3$ | $R_4$ | $R_5$ | phys. Daten |
|---|---|---|---|---|---|---|---|
| 4 | $-N(CH_3)C_4H_9$ | $CF_3$ | Cl | Cl | H | H | |
| 5 | 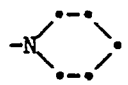 | $CF_3$ | Cl | Cl | H | H | |
| 6 | $-NHCH_3$ | $CF_3$ | Cl | H | Cl | H | |
| 7 | $-N(C_4H_9)_2$ | $CF_3$ | Cl | H | Cl | H | |
| 8 | $-OC_2H_5$ | Cl | Cl | H | Cl | H | |
| 9 | $-OCH_3$ | $CF_3$ | H | H | Cl | H | |
| 10 | $-OC_2H_5$ | $CF_3$ | H | H | Cl | $CH_3$ | |
| 11 | $-N(CH_3)_2$ | $CF_3$ | Cl | H | Cl | $CH_3$ | |
| 12 | 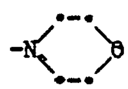 | $CF_3$ | Cl | H | Cl | H | |
| 13 | $-NHCH_3$ | $CF_3$ | Cl | H | H | H | |
| 14 | $-OC_2H_5$ | $CF_3$ | Cl | H | H | H | Sdp. 220°/ 0,03 mb. |
| 15 | $-OC_2H_5$ | $CF_3$ | Cl | H | H | $CH_3$ | |
| 16 | 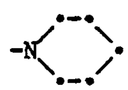 | $CF_3$ | H | H | Cl | H | |
| 17 | $-N(CH_3)_2$ | $CF_3$ | H | H | Cl | $CH_3$ | Smp. 110-113° |
| 18 | $-N(CH_3)_2$ | $CF_3$ | Cl | Cl | H | H | Smp. 108-110° |
| 19 | $-OC_2H_5$ | $CF_3$ | Cl | H | Cl | $CH_3$ | |
| 20 | $-N(CH_3)_2$ | Cl | Cl | H | Cl | H | Sdp. 250°/ 0,2 mb. |

Beispiel 2 : Herstellung eines Stäubemittels

Zur Herstellung eines a) 5 %igen und b) 2 %igen Stäubemittels werden die folgenden Stoffe verwendet :

a) 5 Teile N-[3-(2'-Chlor-4'-trifluormethyl-phenoxy)-6-nitro-phenyl]-N',N'-dimethylformamidin,
95 Teile Talk ;
b) 2 Teile des obigen Wirkstoffes,
1 Teil hochdisperse Kieselsäure,
97 Teile Talk.

Die Wirkstoffe werden mit den Trägerstoffen vermischt und vermahlen.

Anstelle des hier verwendeten Wirkstoffs Nr. 1 kann mit ähnlichem Erfolg einer der Wirkstoffe Nr. 2-20 verwendet werden.

Beispiel 3 : Herstellung einer Paste

Zur Herstellung einer 45 %igen Paste werden folgende Stoffe verwendet :

45 Teile N-[3-(2'-Chlor-4'-trifluormethyl-phenoxy)-6-nitrophenyl]-N',N'-dimethylformamidin oder ein Salz davon,
5 Teile Natriumaluminiumsilikat,
14 Teile Cetylpolyäthylenglykoläther mit 8 Mol Aethylenoxid,

1 Teil Oleylpolyäthylenglykoläther mit 5 Mol Aethylenoxid,
2 Teile Spindelöl,
23 Teile Wasser,
10 Teile Polyäthylenglykol (mittleres Molekulargewicht 400 g/Mol).

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

### Beispiel 4 : Herstellung eines Spritzpulvers

Zur Herstellung eines a) 50 %igen, b) 25 %igen und c) 10 %igen Spritzpulvers werden folgende Bestandteile verwendet :

a) 50 Teile Wirkstoff Nr. 1 oder ein Salz davon,
5 Teile Natriumdibutylnaphthylsulfonat,
3 Teile Naphthalinsulfonsäuren-Phenolsulfonsäuren-Formaldehyd-Kondensat 3 : 2 : 1,
22 Teile Kaolin,
20 Teile Kreide ;

b) 25 Teile des Wirkstoffes Nr. 1,
5 Teile Oelylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin ;

c) 10 Teile eines Salzes des Wirkstoffs Nr. 1,
3 Teile Gemisch der Natriumsalze von gesättigten Fettalkoholen,
5 Teile Naphthalinsulfonsäuren-Formaldehyd-Kondensat,
32 Teile Kreide,
50 Teile Kaolin.

Der Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Anstelle des hier verwendeten Wirkstoffs Nr. 1 kann mit ähnlichem Erfolg einer der Wirkstoffe Nr. 2-20 verwendet werden.

### Beispiel 5 : Herstellung eines Suspensionskonzentrates

Zur Herstellung eines 45 %igen Konzentrats werden folgende Stoffe verwendet :

45 Teile Wirkstoff der Formel I
5 Teile Aethylenglykol,
3 Teile Octylphenoxypolyäthylenglycol mit 9-10 Mol Aethylenoxyd pro Mol Octylphenol,
3 Teile von einem Gemisch aromatischer Sulfonsäuren, kondensiert mit Formaldehyd als Ammoniumsalz,
1 Teil Siliconöl in Form einer 75 %igen Emulsion,
0,1 Teil einer Mischung von 1-(3-Chlorallyl)-3,5,7-triazoazonium-adamantan-chlorid mit Natriumcarbonat, Chloridwert mind. 11,5 %,
0,2 Teile eines biopolymeren Verdickers mit max. 100 Keimen pro Gramm,
42,7 Teile Wasser.

Die Aktivsubstanz wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

### Beispiel 6

Nachweis der Herbizidwirkung vor dem Auflaufen der Pflanzen (Keimhemmung).

Im Gewächshaus werden Pflanzensamen in Blumentöpfen von 12-15 cm Durchmesser gesät. Unmittelbar danach wird die Erdoberfläche mit einer wässrigen Dispersion oder Lösung der Wirkstoffe, erhalten aus einem Emulsionskonzentrat oder Spritzpulver, behandelt. Es werden verschiedene Konzentrationen mit Wirkstoffmengen pro Hektar angewendet. Die Töpfe werden dann im Gewächshaus bei

einer Temperatur von 22-25 °C und 50-70 % relativer Luftfeuchtigkeit gehalten. Nach 3 Wochen wurde der Versuch ausgewertet und der Zustand der Pflanzen gemäss folgender Skala beurteilt:

1 : Pflanzen abgestorben.
2 bis 8 : Zwischenstuffen der Schädigung.
9 : wirkungslos (wie unbehandelte Kontrollpflanzen).
— Pflanze nicht geprüft.
Die Resultate sind in der untenstehenden Tabelle zusammengefasst.

| Verbindung No. | 2 | 14 | 17 | 18 |
|---|---|---|---|---|
| Aufwandmenge in kg/Hektar | 4 2 1 1/2 | 4 2 1 1/2 | 4 2 1 1/2 | 4 2 1 1/2 |
| Pflanze | | | | |
| Weizen | 2 4 7 7 | 7 8 9 9 | 2 6 8 9 | 9 9 9 9 |
| Reis | 2 4 6 7 | 8 9 9 9 | 3 6 8 9 | 9 9 9 9 |
| Soja | 4 6 9 9 | 9 9 9 9 | 8 9 9 9 | 9 9 9 9 |
| Baumwolle | 5 6 7 7 | - - - - | 6 7 9 9 | - - - - |
| avena fatua | 2 2 2 6 | 1 2 4 9 | 2 2 4 8 | 2 6 7 9 |
| alopecurus myosuroides | 1 1 1 2 | 1 1 2 6 | 2 2 4 9 | 2 2 6 9 |
| echinochloa crus galli | 1 1 1 1 | 1 2 3 9 | 1 1 1 2 | 2 3 4 4 |
| rottboellia exaltata | 1 1 2 7 | 2 4 4 7 | 1 1 2 4 | 2 3 3 9 |
| abutilon | 1 1 1 1 | 1 1 8 9 | 1 1 1 3 | 1 1 1 1 |
| chenopodium album | 1 1 1 1 | 1 1 1 1 | 1 1 1 1 | 1 1 1 1 |
| viola tricolor | 1 1 1 1 | 1 1 1 1 | 1 1 1 1 | 1 1 1 1 |

Beispiel 7 : Nachweis der Herbizidwirkung nach Auflaufen der Pflanzen (Kontaktwirkung).

Die Pflanzen, sowohl mono- wie dikotyle Unkräuter und Kulturpflanzen, werden nach dem Auflaufen, im 4- bis 6-Blattstadium mit einer wässrigen Wirkstoffdispersion, in verschiedenen Dosierungen gespritzt. Dann werden sie bei 24° bis 26 °C und 45-60 % relativer Luftfeuchtigkeit im Gewächshaus gehalten. Der Versuch wird nach 15 Tagen beendet und der Zustand der Pflanzen gemäss der im Beispiel 6 gegebenen Skala bewertet. Die Resultate sind in der untenstehenden Tabelle zusammengefasst.

(Siehe Tabelle, Seite 9 f.)

| Verbindung No. | 1 | | | | | 2 | | | | | 14 | | | | 17 | | | | 18 | | | |
|---|---|---|---|---|---|---|---|---|---|---|---|---|---|---|---|---|---|---|---|---|---|---|
| Aufwandmenge kg/Hektar | 2 | 1 | 1/2 | 1/4 | 1/8 | 2 | 1 | 1/2 | 1/4 | 1/8 | 2 | 1 | 1/2 | 1/4 | 4 | 2 | 1 | 1/2 | 2 | 1 | 1/2 | 1/4 |
| Pflanze: | | | | | | | | | | | | | | | | | | | | | | |
| Gerste | 2 | 4 | 7 | 8 | 8 | 2 | 2 | 6 | 8 | 8 | 3 | 6 | 6 | 7 | - | - | - | - | - | - | - | - |
| Weizen | 5 | 9 | 9 | 9 | 9 | 3 | 4 | 6 | 8 | 9 | 3 | 4 | 7 | 8 | 3 | 7 | 7 | 9 | 7 | 8 | 8 | 9 |
| Reis | 6 | 7 | 7 | 9 | 9 | 2 | 6 | 7 | 7 | 8 | 6 | 7 | 7 | 9 | 6 | 7 | 7 | 9 | 9 | 9 | 9 | 9 |
| Soja | 3 | 4 | 4 | 6 | 9 | 3 | 3 | 5 | 7 | 7 | 2 | 3 | 4 | 6 | 4 | 8 | 8 | 9 | 7 | 7 | 9 | 9 |
| avena fatua | 3 | 6 | 9 | 9 | 9 | 2 | 5 | 7 | 9 | 9 | 4 | 4 | 7 | 9 | 3 | 3 | 6 | 7 | 7 | 7 | 7 | 8 |
| alopecurus myos. | 2 | 2 | 3 | 9 | 9 | 1 | 2 | 6 | 7 | 9 | 3 | 3 | 4 | 4 | 3 | 3 | 4 | 4 | 7 | 9 | 9 | 9 |
| echinochloa crus galli | 1 | 1 | 2 | 4 | 4 | 1 | 1 | 2 | 4 | 4 | 2 | 2 | 4 | 6 | 8 | 9 | 9 | 9 | 2 | 2 | 3 | 4 |
| rottboellia exaltata | 4 | 5 | 7 | 8 | 8 | 2 | 3 | 4 | 7 | 8 | 4 | 4 | 6 | 7 | 4 | 5 | 6 | 9 | 7 | 8 | 9 | 9 |
| abutilon | 1 | 1 | 1 | 1 | 1 | 1 | 1 | 1 | 1 | 1 | 1 | 1 | 2 | 2 | 1 | 1 | 1 | 1 | 1 | 2 | 6 | 7 |
| xanthium | - | - | - | - | - | 1 | 1 | 1 | 1 | 1 | 2 | 2 | 3 | 5 | 2 | 3 | 3 | 3 | 1 | 3 | 3 | 4 |
| amaranthus retroflexus | 1 | 1 | 2 | 3 | 3 | 1 | 1 | 1 | 1 | 1 | 1 | 1 | 2 | 2 | - | - | - | - | - | - | - | - |
| chenopodium album | 1 | 1 | 1 | 6 | 6 | 1 | 1 | 1 | 1 | 1 | 1 | 1 | 1 | 1 | 1 | 2 | 3 | 4 | 1 | 3 | 5 | 5 |
| solanum nigrum | 1 | 1 | 1 | 2 | 6 | 1 | 1 | 1 | 2 | 2 | 1 | 1 | 1 | 1 | - | - | - | - | - | - | - | - |
| ipomoea purpurea | 1 | 1 | 1 | 2 | 2 | 1 | 1 | 1 | 1 | 1 | 1 | 1 | 1 | 1 | 1 | 2 | 2 | 3 | 2 | 2 | 2 | 3 |
| sinapis alba | 1 | 1 | 1 | 1 | 3 | 1 | 1 | 1 | 1 | 1 | 1 | 1 | 3 | 3 | 1 | 1 | 1 | 1 | 1 | 1 | 2 | 2 |
| chrysanthemum leucum | 1 | 1 | 1 | 1 | 1 | 1 | 1 | 1 | 1 | 1 | 1 | 1 | 2 | 3 | - | - | - | - | - | - | - | - |
| galium aparine | 1 | 1 | 1 | 1 | 1 | 1 | 1 | 2 | 2 | 3 | 1 | 1 | 1 | 2 | 2 | 2 | 3 | 7 | 3 | 3 | 3 | 4 |
| viola tricolor | 1 | 1 | 1 | 1 | 1 | 1 | 1 | 1 | 1 | 1 | 1 | 1 | 1 | 2 | 1 | 1 | 1 | 1 | 1 | 2 | 2 | 2 |
| veronica sp | 1 | 1 | 1 | 1 | 1 | 1 | 1 | 1 | 1 | 1 | 1 | 1 | 1 | 1 | - | - | - | - | - | - | - | - |

Die geprüften Verbindungen der Formel I zeigen schon bei geringen Aufwandmengen gegen die dikotylen, aber auch einige monokotyle Unkräuter starke herbizide Wirkung, ohne dass die Kulturpflanzen, wie zum Beispiel Weizen und Reis nennenswert geschädigt werden.

Beispiel 8 : Nachweis der selektiven herbiziden Wirkung auf Reis um Nachauflaufverfahren

Reispflänzchen, welche 25 Tage alt sind, wurden im Gewächshaus in grosse rechteckige Eternitschalten verpflanzt. Zwischen die Reihen der Reispflanzen wurden dann Samen der in Reiskulturen vorkommenden Unkräuter Echinochloa crus galli und Cyperus difformis gesät. Die Schalen wurden gut bewässert und bei einer Temperatur von ca. 25 °C und hoher Luftfeuchtigkeit gehalten. Nach 12 Tagen, wenn die Unkräuter aufgelaufen sind und das 2-3 Blattstadium erreicht haben, wurde die Erde in der Schale mit einer 2,5 cm hohen Schicht Wasser bedeckt. Der Wirkstoff wurde dann als Emulsionskonzentrat mittels einer Pipette zwischen die Pflanzenreihen appliziert, wobei man das Emulsionskonzentrat so verdünnte und auftrug, dass es einer Applikationsmenge im Feld von 4, 2, 1 und 1/2 kg Wirkstoff pro Hektar entsprach. Der Versuch wurde nach 4 Wochen ausgewertet. Die Resultate sind in der untenstehenden Tabelle zusammengefasst.

| Test Verbindung | 1 | 2 | 14 | 17 | 18 | 21 |
|---|---|---|---|---|---|---|
| Aufwandmenge kg/ha | 4 2 1 1/2 | 4 2 1 1/2 | 4 2 1 1/2 | 4 2 1 1/2 | 4 2 1 1/2 | 4 2 |
| Pflanze: | | | | | | |
| Reis | 5 6 6 7 | 3 3 4 5 | 5 6 7 8 | 5 5 6 6 | 7 8 9 9 | 6 7 |
| Echinochloa crus galli | 4 4 5 7 | 1 1 1 1 | 1 1 2 5 | 1 1 1 1 | 4 5 6 7 | 3 6 |
| cyperus difformis | 1 1 2 3 | 1 1 1 1 | 1 1 1 2 | 3 3 4 4 | 2 3 4 6 | 2 4 |

Beispiel 9 : Nachweis der Desikkations- und Defoliationswirkung auf Baumwolle

In einem Gewächshaus wurden Baumwollpflanzen der Sorte Deltapine in Tontöpfen angezogen. Nach abgeschlossener Kapselbildung wurden sie mit wässerigen Zubereitungen der Wirkstoffe No. 2 und 14 in einer Aufwandmenge, die 1, 2, 0,6 und 0,3 kg/ha im Feld entspräche, gespritzt. Unbehandelte Pflanzen wurden als Kontrolle belassen. Die Auswertung des Versuches erfolgte 14 Tage nach Applikation der Wirksubstanz durch Bestimmen des Grades an Defoliation (% abgefallene Blätter) und an Desikkation (% Austrocknung der an der Pflanze verbleibenden Blätter. 100 % = alle Blätter angetrocknet oder abgefallen).

Die Resultate sind in der untenstehenden Tabelle zusammengefasst.

| | Defoliation | | | Desikkation | | |
|---|---|---|---|---|---|---|
| Aufwandmenge in kg/ha | 0,3 | 0,6 | 1,2 | 0,3 | 0,6 | 1,2 |
| Verbindung No. | | | | | | |
| 2 | 50% | 50% | 30% | 80% | 90% | 80% |
| 14 | 80% | 30% | 40% | 90% | 90% | 90% |

Beispiel 10 : Nachweis der Wuchshemmung bei Gräsern

Im Gewächshaus werden in Kunststoffschalen mit Erde-Torf-Sand-Gemisch (6 : 3 : 1) Samen von Folium perenne, poa pratensis, festuca ovina und dactylis glomerata, cynodon, Gerste und Roggen ausgesät und normal bewässert. Die aufgelaufenen Gräser wurden wöchentlich bis auf 4 cam Höhe zurückgeschnitten und 40 Tage nach der Aussaat und 1 Tag nach dem letzten Schnitt mit wässerigen Spritzbrühen der Verbindung No. 17 behandelt. Die Wirkstoffmenge betrug umgerechnet 0,5 kg und 2,5 kg Aktivsubstanz pro Hektar. Das Wachstum der Gräser wird 21 Tage nach Applikation mit derjenigen einer unbehandelten Kontrolle verglichen und der Neuzuwachs in % des Wuchses von unbehandelten Kontrollgräsern (= 100 %) angegeben. Die Resultate sind untenstehend zusammengefasst.

| Pflanze | lolium | poa | festucca | dactylis | cynodon | Gerste | Roggen |
|---|---|---|---|---|---|---|---|
| Aufwandmenge kg/ha | | | | | | | |
| 0;5 | 86% | 65% | 81% | 79% | - | 66% | 42% |
| 2,5 | 57% | 19% | 37% | 36% | 26% | 47% | 55% |

**Ansprüche**

1. 3-Phenoxy-methylenaniline der allgemeinen Formel I

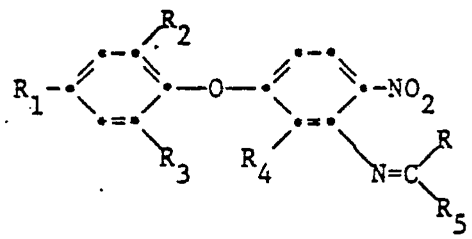

(I)

worin

R einen Rest

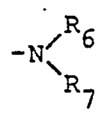

oder $C_1$-$C_4$-Alkoxy,

$R_1$ Halogen oder $C_1$-$C_2$-Halogenalkyl,

$R_2$, $R_3$ und $R_4$ unabhängig voneinander je Wasserstoff oder Halogen,

$R_5$ Wasserstoff oder $C_1$-$C_4$-Alkyl,

$R_6$ Wasserstoff, $C_1$-$C_4$-Alkyl, $C_2$-$C_4$-Alkenyl oder $C_2$-$C_4$-Alkinyl und

$R_7$ Wasserstoff oder $C_1$-$C_4$-Alkyl bedeutet, wobei die Reste $R_5$ und $R_6$ oder $R_6$ und $R_7$ zusammen mit dem Stickstoffatom einen gegebenenfalls durch weitere Heteroatome unterbrochenen Heterocyclus bilden können, sowie die Salze mit Säuren wenn R einen Aminrest bedeutet.

2. 3-Phenoxy-methylenaniline der Formel I, gemäss Anspruch 1, dadurch gekennzeichnet, dass R den Aminorest $—NR_6R_7$ bedeutet.

3. Verbindungen der Formel I, gemäss Anspruch 1, dadurch gekennzeichnet, dass der Rest $R_1$ eine Trifluormethylgruppe bedeutet.

4. Verbindungen der Formel I, gemäss Anspruch 1, dadurch gekennzeichnet, dass von den Resten $R_2$, $R_3$ und $R_4$ unabhängig voneinander jeweils 1 bis 2 Reste Chloratome darstellen und die übrigen Wasserstoff bedeuten.

5. Verbindungen der Formel I, gemäss Ansprüchen 3 oder 4, dadurch gekennzeichnet, dass $R_1$ Trifluormethyl, $R_2$ Chlor und $R_3$ und $R_4$ Wasserstoff oder einer der Reste $R_3$ und $R_4$ Chlor und der andere Wasserstoff bedeutet.

6. 3-Phenoxy-methylenaniline der Formel I, gemäss Ansprüchen 4 oder 5, dadurch gekennzeichnet, dass $R_1$ Trifluormethyl, $R_2$ Chlor, $R_5$ Wasserstoff und $R_3$ und $R_4$ Wasserstoff oder einer der Reste $R_3$ und $R_4$ Chlor und der andere Wasserstoff bedeutet.

7. N-[3-(2'-Chlor-4'-trifluormethylphenoxy]-6-nitrophenyl]-N',N'-dimethylformamidin gemäss Anspruch 1.

8. N-[3-(2'-Chlor-4-trifluormethyl-phenoxy)-6-nitrophenyl]-N',N'-dimethyl-acetamidin gemäss Anspruch 1.

9. N-[1-Chlor-3-(2'-chlor-4'-trifluormethylphenoxy)-6-nitrophenyl]-N',N'-dimethyl-formamidin gemäss Anspruch 1.

10. N-[3-(2',6'-Dichlor-4-trifluormethylphenoxy)-6-nitro-phenyl]-N',N'-dimethylformamidin gemäss Anspruch 1.

11. N-[3-(2',6'-Dichlor-4-trifluormethylphenoxy)-6-nitro-phenyl]-N',N'-dimethylacetamidin gemäss Anspruch 1.

12. N-[2-Chlor-3-(2'-chlor-4'-trifluormethylphenoxy)-6-nitrophenyl]-N',N'-dimethyl-acetamidin gemäss Anspruch 1.

13. N-[3-(2'-chlor-4'-trifluormethyl-phenoxy)-6-nitrophenyl]-äthoxy-acetimid gemäss Anspruch 1.

14. Verfahren zur Herstellung von Verbindungen der Formel I, in denen R eine Aminogruppe $-NR_6R_7$ ist, dadurch gekennzeichnet, dass man ein Anilin der Formel II

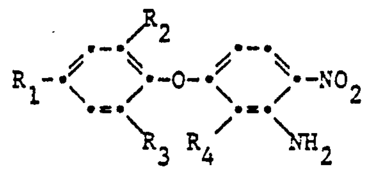

(II)

in Gegenwart eines Kondensationsmittels und gegebenenfalls einer Base mit einem Carbamid der Formel III

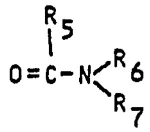

(III)

worin $R_1$ bis $R_7$ die unter Formel I, Anspruch 1, gegebene Bedeutung haben, umsetzt und gegebenenfalls in die Salze überführt.

15. Verfahren zur Herstellung von Verbindungen der Formel I, in denen R eine Aminogruppe $-NR_6R_7$ ist, dadurch gekennzeichnet, dass man ein Anilin der Formel II mit einem Carbamiddiacetal der Formel IV

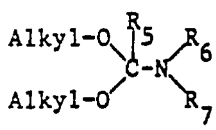

(IV)

worin $R_5$ bis $R_7$ die unter Formel I gegebene Bedeutung haben und Alkyl geradkettiges $C_1$-$C_4$-Alkyl bedeutet, umsetzt und gegebenenfalls in die Salze überführt.

16. Verfahren zur Herstellung von Verbindungen der Formel I, in denen R eine $C_1$-$C_4$-Alkoxygruppe ist, dadurch gekennzeichnet, dass man ein Anilin der Formel II mit einem Orthoester der Formel V

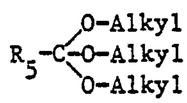

(V)

umsetzt, worin $R_5$ die unter Formel I gegebene Bedeutung hat und Alkyl geradkettiges $C_1$-$C_4$-Alkyl bedeutet.

17. Verfahren zur Herstellung von Verbindungen der Formel I, in denen R eine Aminogruppe $-NR_6R_7$ ist, dadurch gekennzeichnet, dass man eine Verbindung der Formel I, in der R eine Alkoxygruppe bedeutet, mit einem Amin der Formel VI

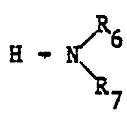

(VI)

umsetzt, worin $R_6$ und $R_7$ die unter Formel I gegebene Bedeutung haben.

18. Verfahren zur Herstellung der Salze der Verbindungen der Formel I, in denen R eine Aminogruppe $-NR_6R_7$ ist, dadurch gekennzeichnet, dass man ein Amidin der Formel I in einem inerten Lösungsmittel löst, mit äquimolaren Mengen Säure umsetzt und das Lösungsmittel abdampft.

19. Herbizides Mittel, dadurch gekennzeichnet, dass es neben Träger- und/oder anderen Zuschlagstoffen als Wirkstoff mindestens eine Verbindung der Formel I, gemäss Anspruch 1, enthält.

20. Die Verwendung der 3-Phenoxy-methylenaniline der Formel I, Anspruch 1, oder sie enthaltende Mittel, zur Bekämpfung von Unkräutern.

21. Die Verwendung der 3-Phenoxy-methylenaniline der Formel I, Anspruch 1, oder sie enthaltende Mittel zur selektiven Bekämpfung von Unkräutern in Nutzpflanzenkulturen.

22. Die Verwendung der 3-Phenoxy-methylenaniline der Formel I, Anspruch 1 oder sie enthaltende Mittel zur post-emergenten Bekämpfung von Unkräutern in Kulturen von Getreide, Reis und Soja.

**Claims**

1. A 3-phenoxymethylene-aniline of the general formula I

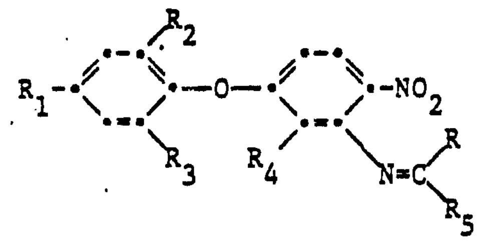

(I)

wherein

R is a

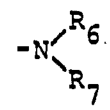

radical or $C_1$-$C_4$ alkoxy,

$R_1$ is halogen or $C_1$-$C_2$ haloalkyl,

$R_2$, $R_3$ and $R_4$, each independently of the other, are hydrogen or halogen,

$R_5$ is hydrogen or $C_1$-$C_4$ alkyl,

$R_6$ is hydrogen, $C_1$-$C_4$ alkyl, $C_2$-$C_4$ alkenyl or $C_2$-$C_4$ alkynyl, and

$R_7$ is hydrogen or $C_1$-$C_4$ alkyl, and $R_5$ and $R_6$, or $R_6$ and $R_7$, together with the nitrogen atom to which they are attached, can form a heterocyclic ring system which may be interrupted by further hetero-atoms, or a salt thereof with an acid if R is an amino group.

2. A 3-phenoxymethylene-aniline of the formula I according to claim 1, wherein R is the amino group —$NR_6R_7$.

3. A compound of the formula I according to claim 1, wherein $R_1$ is a trifluoromethyl group.

4. A compound of the formula I according to claim 1, wherein one to two of $R_2$, $R_3$ and $R_4$, each independently of the other, are 1 to 2 chlorine atoms and the others are hydrogen.

5. A compound of the formula I according to either of claims 3 or 4, wherein $R_1$ is trifluoromethyl, $R_2$ is chlorine, and $R_3$ and $R_4$ are hydrogen, or one of $R_3$ and $R_4$ is chlorine and the other is hydrogen.

6. A 3-phenoxymethylene-aniline of the formula I according to either of claims 4 or 5, wherein $R_1$ is trifluoromethyl, $R_2$ is chlorine, $R_5$ is hydrogen, and $R_3$ and $R_4$ are hydrogen, or one of $R_3$ and $R_4$ is chlorine and the other is hydrogen.

7. N-[3-(2′-Chloro-4′-trifluoromethylphenoxy]-6-nitrophenyl]-N′,N′-dimethylformamidine according to claim 1.

8. N-[3-(2′-Chloro-4-trifluoromethylphenoxy)-6-nitrophenyl]-N′,N′-dimethylacetamidine according to claim 1.

9. N-[2-Chloro-3-(2′-chloro-4′-trifluoromethylphenoxy)-6-nitrophenyl]-N′,N′-dimethylformamidine according to claim 1.

10. N-[3-(2′,6′-Dichloro-4-trifluoromethylphenoxy)-6-nitrophenyl]-N′,N′-dimethylformamidine according to claim 1.

11. N-[3-(2′,6′-Dichloro-4-trifluoromethylphenoxy)-6-nitrophenyl]-N′,N′-dimethylacetamidine according to claim 1.

12. N-[2-Chloro-3-(2′-chloro-4′-trifluoromethylphenoxy)-6-nitrophenyl]-N′,N′-dimethylacetamidine according to claim 1.

13. N-[3-(2′-Chloro-4′-trifluoromethylphenoxy)-6-nitrophenyl]-ethoxyacetimide according to claim 1.

14. A process for the production of a compound of the formula I, wherein R is an amino group —$NR_6R_7$, which process comprises reacting an aniline of the formula II

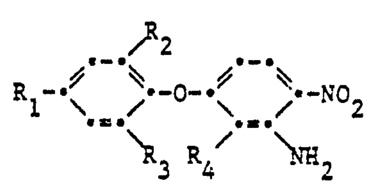

(II)

with a carbamide of the formula III

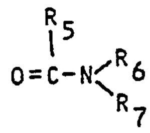

(III)

in which formulae $R_1$ to $R_7$ are as defined for formula I in claim 1, in the presence of a condensing agent and optionally of a base, and, if desired, converting said compound of the formula I into a salt thereof.

15. A process for the production of a compound of the formula I, wherein R is an amino group $—NR_6R_7$, which process comprises reacting an aniline of the formula II with a carbamide diacetal of the formula IV

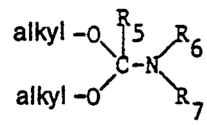

(IV)

wherein $R_5$ to $R_7$ are as defined for formula I and alkyl denotes $C_1$-$C_4$ alkyl, and, if desired, converting said compound of formula I into a salt thereof.

16. A process for the production of a compound of the formula I, wherein R is a $C_1$-$C_4$ alkoxy group, which process comprises reacting an aniline of the formula II with an ortho-ester of the formula V

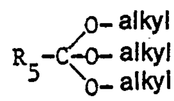

(V)

wherein $R_5$ is as defined for formula I and alkyl denotes straight-chain $C_1$-$C_4$ alkyl.

17. A process for the production of a compound of the formula I, wherein R is an amino group $—NR_6R_7$, which process comprises reacting a compound of the formula I, in which R is an alkoxy group, with an amine of the formula VI

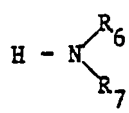

(VI)

wherein $R_6$ and $R_7$ are as defined for formula I.

18. A process for the production of a salt of a compound of the formula I, wherein R is an amino group $—NR_6R_7$, which process comprises dissolving an amidine of the formula I in an inert solvent, reacting it with the equimolar amount of an acid, and removing the solvent by evaporation.

19. A herbicidal composition which contains at least one compound of the formula I according to claim 1, together with carriers and/or other adjuvants.

20. A method of controlling weeds at a locus, which method comprises applying to said locus a herbicidally effective amount of a 3-phenoxy-methylene-aniline of the formula I according to claim 1 or of a composition containing such a compound.

21. A method of selectively controlling weeds in crops of useful plants, which comprises applying to said crops a herbicidally effective amount of a 3-phenoxymethylene-aniline of the formula I according to claim 1 or of a composition containing such a compound.

22. A method of controlling weeds postemergence in crops of cereals, rice and soybeans, which method comprises applying to said crops a herbicidally effective amount of a 3-phenoxymethylene-aniline of the formula I according to claim 1 or of a composition containing such a compound.

**Revendications**

1. 3-phénoxy-méthylanilines de formule générale I :

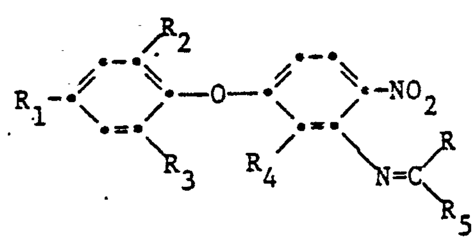

(I)

où R représente un radical

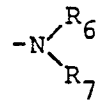

ou un alcoxy en $C_1$ à $C_4$,

$R_1$ représente un halogène ou un halogènealcoyle en $C_1$ à $C_2$,

$R_2$, $R_3$ et $R_4$ représentent chacun indépendamment l'un de l'autre un hydrogène ou un halogène,

$R_5$ représente un hydrogène ou un alcoyle en $C_1$ à $C_4$,

$R_6$ représente un hydrogène, un alcoyle en $C_1$ à $C_4$, un alcényle en $C_2$ à $C_4$ ou un alcynyle en $C_2$ à $C_4$, et

$R_7$ représente un hydrogène ou un alcoyle en $C_1$ à $C_4$, où les radicaux $R_5$ et $R_6$ ou $R_6$ et $R_7$ peuvent former ensemble avec l'atome d'azote un hétérocycle éventuellement interrompu par d'autres hétéroatomes, ainsi que les sels formés avec des acides lorsque R représente un radical amine.

2. 3-phénoxy-méthylèneanilines de formule I selon la revendication 1, caractérisées en ce que R représente le radical amino $—NR_6R_7$.

3. Composés de formule I selon la revendication 1, caractérisés en ce que le radical $R_1$ représente un groupe trifluorométhyle.

4. Composés de formule I selon la revendication 1, caractérisés en ce que, parmi les radicaux $R_2$, $R_3$ et $R_4$, indépendamment l'un de l'autre, à chaque fois de 1 à 2 radicaux représentent des atomes de chlore et les autres représentent un hydrogène.

5. Composés de formule I selon la revendication 3 ou 4, caractérisés en ce que $R_1$ représente un trifluorométhyle, $R_2$ un chlore et $R_3$ et $R_4$ un hydrogène, ou bien où l'un des radicaux $R_3$ et $R_4$ représente un chlore et l'autre représente un hydrogène.

6. 3-phénoxy-méthylèneanilines de formule I selon la revendication 4 ou 5, caractérisées en ce que $R_1$ représente un trifluorométhyle, $R_2$ représente un chlore, $R_5$ représente un hydrogène et $R_3$ et $R_4$ un hydrogène, ou bien où l'un des radicaux $R_3$ et $R_4$ représente un chlore et l'autre un hydrogène.

7. N-[3-(2'-chloro-4'-trifluorométhylphénoxy)-6-nitrophényl]-N',N'-diméthylformamidine selon la revendication 1.

8. N-[3-(2'-chloro-4-trifluorométhylphénoxy)-6-nitrophényl]-N',N'-diméthyl-acétamidine selon la revendication 1.

9. N-[2-chloro-3-(2'-chloro-4'-trifluorométhylphénoxy)-6-nitrophényl-N',N'-diméthyl-formamidine selon la revendication 1.

10. N-[3-(2',6'-dichloro-4-trifluorométhylphénoxy)-6-nitro-phényl]-N',N'-diméthylformamidine selon la revendication 1.

11. N-[3-(2',6'-dichloro-4-trifluorométhylphénoxy)-6-nitro-phényl]-N',N'-diméthylacétamidine selon la revendication 1.

12. N-[2-chloro-3-(2'-chloro-4'-trifluorométhylphénoxy)-6-nitrophényl]-N',N'-diméthyl-acétamidine selon la revendication 1.

13. N-[3-(2'-chloro-4'-trifluorométhylphénoxy)-6-nitrophényl]-éthoxy-acétimide selon la revendication 1.

14. Procédé de préparation de composés de formule I où R est un groupe amino $—NR_6R_7$, caractérisé en ce qu'on fait réagir une aniline de formule II :

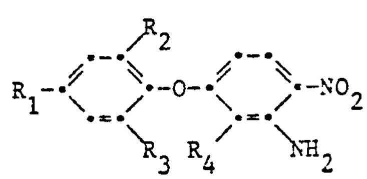

(II)

en présence d'un agent de condensation et éventuellement d'une base avec un carbamide de formule III :

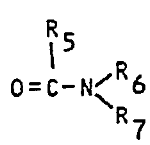

(III)

où $R_1$ à $R_7$ ont la signification donnée pour la formule I de la revendication 1 et éventuellement en ce qu'on transforme en les sels.

15. Procédé de préparation de composés de formule I, où R est un groupe amino $—NR_6R_7$, caractérisé en ce qu'on fait réagir une aniline de formule II avec un carbamidediacétal de formule IV :

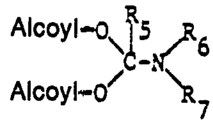 (IV)

où $R_5$ à $R_7$ ont la signification donnée pour la formule I et alcoyle représente un alcoyle en $C_1$ à $C_4$ à chaîne droite, et éventuellement en ce qu'on procède à une salification.

16. Procédé de préparation de composés de formule I, où R est un groupe alcoxy en $C_1$ à $C_4$, caractérisé en ce qu'on fait réagir une aniline de formule II avec un orthoester de formule V :

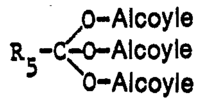 (V)

où $R_5$ a la signification donnée pour la formule I et alcoyle représente un alcoyle en $C_1$ à $C_4$ à chaîne droite.

17. Procédé de préparation de composés de formule I où R représente un groupe amino $-NR_6R_7$, caractérisé en ce qu'on fait réagir un composé de formule I où R représente un groupe alcoxy, avec une amine de formule VI :

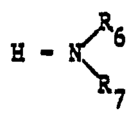 (VI)

où $R_6$ et $R_7$ ont la signification donnée pour la formule I.

18. Procédé de préparation des sels des composés de formule I où R représente un groupe amino $-NR_6R_7$, caractérisé en ce qu'on dissout une amidine de formule I dans un solvant inerte, en ce qu'on la fait réagir avec des quantités équimolaires d'acide et en ce qu'on fait évaporer le solvant.

19. Agent herbicide caractérisé en ce qu'il contient outre des supports et/ou autres additifs, comme matière active, au moins un composé de formule I selon la revendication 1.

20. Application des 3-phénoxy-méthylèneanilines de formule I de la revendication 1 ou des agents qui les contiennent à la lutte contre les mauvaises herbes.

21. Application des 3-phénoxy-méthylèneanilines de formule I de la revendication 1 ou des agents qui les contiennent à la lutte sélective contre les mauvaises herbes dans les cultures de plantes utiles.

22. Application des 3-phénoxy-méthylèneanilines de formule I de la revendication 1 ou des agents qui les contiennent à la lutte en post-levée contre les mauvaises herbes dans les cultures de céréales, de riz et de soja.